# EUROPEAN PATENT APPLICATION

(11) **EP 3 985 093 A1**
(43) Date of publication of application: **20.04.2022**
(21) Application number: 21202791.6
(22) Date of filing: 15.10.2021
(51) Int. Cl.: C12M 1/00, A01N 1/02, A61B 10/00, C12M 3/00, C12M 1/12

(54) **TRANSFER SYSTEM FOR BIOLOGICAL SAMPLES**

(30) Priority: 15.10.2020 IT 202000024310
(71) Applicant: Bioair S.p.A, 20145 Milano (IT)
(72) Inventor: SEVERINA, Franco, I-20142 Milano (IT); ZANINI, Cristina, I-20142 Milano (IT); BONIFACIO, Marco, I-20142 Milano (IT)
(74) Representative: Croce, Valeria

(57) **Abstract**

A transfer system (1) for biological samples (2), in particular for transferring biological samples from a Grade A isolator (ISO 4.8) to standard external CO₂ incubators, and vice versa, without breaking the isolation continuity of the biological samples, comprising an RTP container (3) defining a container opening (11) which is closable by a container lid (12) provided with a HEPA or ULPA filter, and an auxiliary container opening (15) which is closable by an auxiliary container lid (16) provided with a HEPA or ULPA filter, and opposite to the container opening 11, and a gyroscopic flask-holder (4), wherein the gyroscopic flask-holder (4) is housed inside the RTP container (3).

## Description

The present invention relates to a transfer system for biological samples, in particular for transferring biological samples from a Grade A isolator (ISO 4.8) to standard external CO₂ incubators, and vice versa, without breaking the isolation continuity of the biological samples.

The need is felt to have systems for transferring biological samples without breaking the isolation continuity, for applications concerning, for example, advanced cell therapies, the production of ATMPs in GMP (Advanced Therapy Medicinal Products in Good Manufacturing Practice), regenerative medicine, gene therapies.

Transfer systems for biological samples are known, such as integrated CO₂ incubators or with docking system.

Prior art document US 2012/077220 describes a patient-specific container designed to contain a biological sample taken from a single patient, previously processed in an isolator, to which it is hermetically connectable through appropriate connection means, which ensure the sterility thereof. The container includes two further openings, provided with filters, which act as an inlet and an outlet when connected to a network of pipes containing fluids, e.g., gas, for the correct maintenance and the correct growth of the biological sample.

Prior art document EP 2 311 935 describes a transport device for material of tissue origin comprising a gyroscopic system, adapted to reduce vibrations to the tissue during transport, and a temperature control system.

Prior art document WO 2016/183513 describes a device adapted to transport cell cultures, inserted into the device only inside an isolator to avoid contamination, and adapted to maintain the correct environmental conditions for cell growth and proliferation, by virtue of two openings provided with filters which ensure the correct gas exchange when the device is housed in cell growth incubators.

However, such systems are not always sufficient for the purpose, they do not always ensure high isolation standards, are expensive and complex to use.

The object of the present invention is to provide a transfer system for biological samples, in particular for transferring biological samples from a Grade A isolator (ISO 4.8) to standard external CO₂ incubators, and vice versa, without breaking the isolation continuity of the biological samples, which solves at least some of the problems highlighted in the prior art.

A particular object of the present invention is to provide a transfer system for biological samples which eliminates the risk of contamination during the transfer from the Grade A isolator to the standard external CO₂ incubators, which eliminates the risk of cross contamination between biological samples from different patients, and which allows the growth of the cells of the biological samples inside standard CO₂ incubators outside the Grade A isolator.

It is a further particular object of the present invention to provide a transfer system for biological samples with low costs and complexity.

These and other objects are achieved by a transfer system for biological samples, in particular for transferring biological samples from a Grade A isolator (ISO 4.8) to standard external CO₂ incubators, and vice versa, without breaking the isolation continuity of the biological samples, according to claim 1.

For the purposes of the present invention, Grade A isolators (ISO 4.8), standard external CO₂ incubators, and the transfer system for biological samples are to be understood as distinct devices.

In particular, each of the Grade A isolators, the standard external CO₂ incubators, and the transfer system for biological samples is designated to perform the specific function(s), known to those skilled in the art.

The dependent claims relate to preferred and advantageous embodiments of the present invention.

In order to better understand the invention and appreciate the advantages thereof, some non-limiting exemplary embodiments thereof will be described below with reference to the accompanying drawings, in which:
- figure 1 is a front view of a transfer system for biological samples, according to an embodiment of the invention;
- figure 2 is a longitudinal sectional view of a transfer system for biological samples, according to an embodiment of the invention;
- figure 3 is a cross-sectional view of a transfer system for biological samples, inserted into a standard external CO₂ incubator according to an embodiment of the invention;
- figure 4 is a perspective view of a transfer system for biological samples, according to an embodiment of the invention;
- figure 5A is a partial view of a transfer system for biological samples, according to an embodiment of the invention, in a first operating configuration;
- figure 5B is a partial view of a transfer system for biological samples, according to an embodiment of the invention, in a second operating configuration;
- figure 5C is a partial view of a transfer system for biological samples, according to an embodiment of the invention, in a third operating configuration;
- figure 6 is a detailed longitudinal sectional view of a transfer system for biological samples, according to an embodiment of the invention;
- figure 7A is a detailed longitudinal sectional view of a transfer system for biological samples, according to an embodiment of the invention;
- figure 7B is a longitudinal sectional view of the system depicted in figure 7A, in a first operating step;
- figure 8A is a further detailed longitudinal sectional view of the system depicted in figure 7A;
- figure 8B is a longitudinal sectional view of the system depicted in figure 8A, in a second operating step;
- figure 9A is a further detailed longitudinal sectional view of the system depicted in figure 7A;
- figure 9B is a longitudinal sectional view of the system depicted in figure 9A, in a third operating step;
- figure 10 is a side view of a transfer system for biological samples, according to an embodiment of the invention;
- figure 11 is a perspective view of a component of a transfer system for biological samples, according to an embodiment of the invention;
- figure 12 is a perspective view, showing externally visible and invisible details, of a transfer system for biological samples, according to an embodiment of the invention;
- figure 13 is a longitudinal sectional view of a transfer system for biological samples, according to an embodiment of the invention;

With reference to the figures, a transfer system for biological samples 2 is generally indicated by the reference numeral 1.

The transfer system 1 for biological samples 2 comprises an RTP container 3.

The RTP container 3 defines a container opening 11 which is closable by a container lid 12.

Furthermore, the transfer system 1 for biological samples 2 comprises a gyroscopic flask-holder 4 housed inside the RTP container 3.

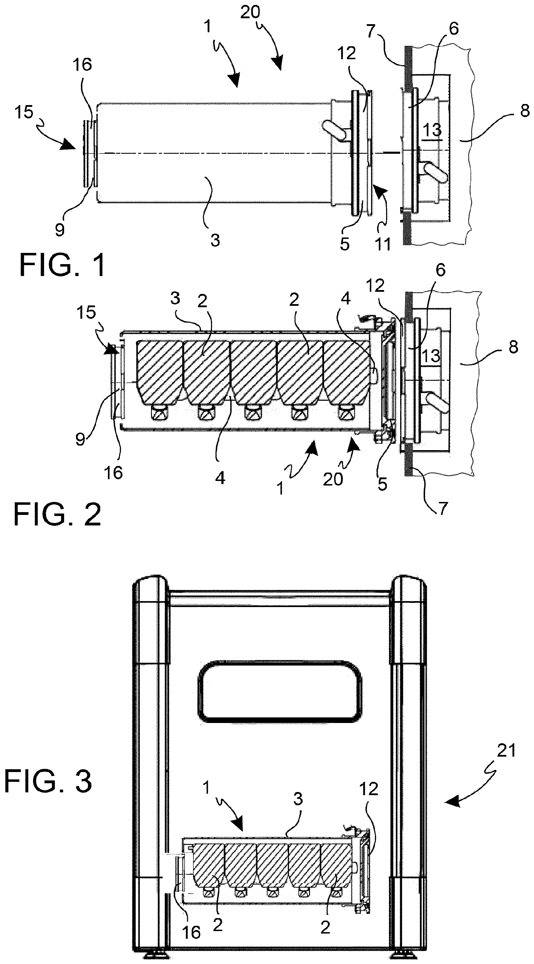

Advantageously, a system 1 thus configured reduces the risk of contamination, for example of cross contamination between biological samples from different patients (figs. 1, 2 and 4).

With further advantage, the system 1 has low costs and complexity.

"RTP" means "Rapid Transfer Port" technology, otherwise known as DPTE^{®}.

A "gyroscopic" flask-holder 4 means a flask-holder hinged to the RTP container 3 about a rotation axis, so as to preserve the orientation thereof with respect to the rotation axis, regardless of any rotation of the RTP container 3 with respect to the rotation axis.

Advantageously, this allows preserving the biological samples 2 supported by the gyroscopic flask-holder 4 in a predetermined orientation, avoiding the risk of overturning the biological samples 2 in case of a rotation of the RTP container 3.

According to an embodiment, the RTP container 3 comprises an auxiliary container opening 15 which is closable by an auxiliary container lid 16 and opposite to the container opening 11 (figs. 1, 2).

Preferably, the auxiliary container opening 15 is of a different diameter than the container opening 11.

Advantageously, this makes the ventilation of the interior of the system 1 more efficient when inserted into a standard external CO₂ incubator.

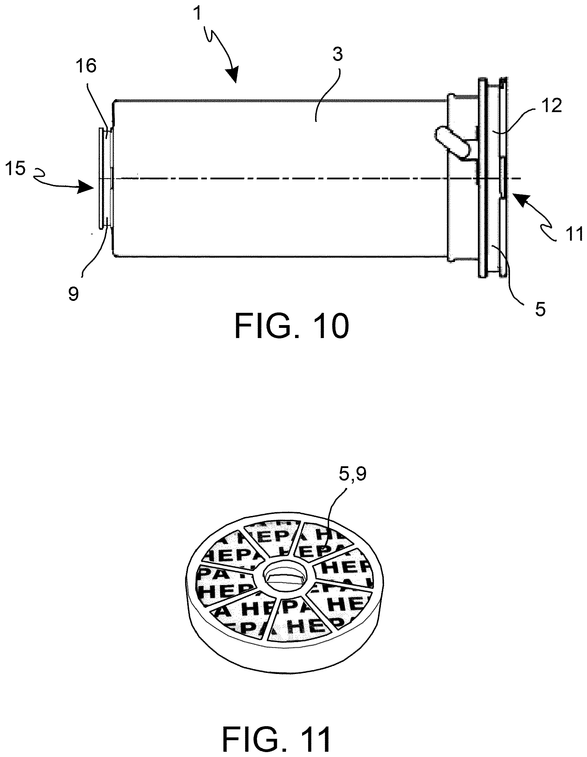

According to an aspect of the present invention, the system 1 further comprises a HEPA (H14) or ULPA (U15) filter 5, positioned at the container lid 12 (fig. 11).

Such a HEPA or ULPA filter 5 has the following functions:
- protecting the biological samples from external contamination during the transfer from the Grade A isolator to the standard external CO₂ incubators;
- protecting the biological samples from cross contamination when the container is placed inside standard external CO₂ incubators;
- allowing the gas exchange between the transfer system 1 for biological samples closed by the lid 12 and the environment inside the standard external CO₂ incubators, for the growth of the cells of the biological samples.

Advantageously, such a HEPA or ULPA filter 5 connected to the container lid 12 avoids the risk of contamination of the interior of the RTP container 3 when the RTP container 3 is disconnected from the Grade A isolator (ISO 4.8) to be transferred to a standard external CO₂ incubator, for example, through a non-sterile environment such as the room where the Grade A isolator (ISO 4.8) and standard external CO₂ incubators are placed.

With a further advantage, such a HEPA or ULPA filter 5 connected to the container lid 12 ensures excellent gas permeability when the container is inserted into a standard external CO₂ incubator.

The gas exchange between the transfer system 1 for biological samples and the controlled environment inside the standard external CO₂ incubator occurs only by virtue of the presence of such a filter 5.

Advantageously, the transfer system 1 for biological samples can simply be placed inside the standard external CO₂ incubators, without including connection systems between the standard external CO₂ incubators and the transfer system 1 for biological samples.

With a further advantage, it is possible to transfer a plurality of transfer systems 1 for biological samples inside a single standard external CO₂ incubator, even containing biological samples from different patients, thus considerably increasing the number of samples which are insertable into the same incubator.

According to an embodiment of the invention, the system 1 comprises an auxiliary HEPA or ULPA filter 9 positioned at the auxiliary container lid 16 for the optimization of the gas circulation when the transfer container is placed inside standard external CO₂ incubators (fig. 10).

Such an auxiliary HEPA or ULPA filter 9 has the following functions:
- protecting the biological samples from external contamination during the transfer from the Grade A isolator to the standard external CO₂ incubators;
- protecting the biological samples from cross contamination when the container is placed inside standard external CO₂ incubators;
- allowing the gas exchange between the transfer system 1 for biological samples closed by the lid 12 and the auxiliary lid 16 and the environment inside the standard external CO₂ incubators for the growth of the cells of the biological samples, thus optimizing the circulation of such gases inside the RTP containers 3 in synergy with the filter 5.

According to a preferred embodiment, either filter 5 or auxiliary filter 9, or both, is/are a HEPA H14 filter, or a ULPA U15 filter.

Advantageously, this type of filter ensures minimum contamination levels and ensures excellent gas permeability, when the container is inserted into a standard external CO₂ incubator.

According to an aspect of the present invention, the system 1 comprises an RTP door 6 associated with a door flange 7 of a Grade A isolator 8 (figs. 1, 2, 5A-5C).

The door flange 7 defines a door opening 13 which is closable by the RTP door 6.

The container lid 12 and the RTP door 6 are configured to geometrically connect to each other, so that the geometrically connected container lid 12 and RTP door 6 are jointly separable from the door flange 7.

Advantageously, such a configuration drastically reduces the contamination risk of the biological samples 2, since any contaminated area remains confined between the container lid 12 and the RTP door 6.

According to an embodiment of the invention, the RTP container 3 comprises a container seal 10 positioned at the container lid 12.

Furthermore, the RTP door 6 comprises a door seal 22.

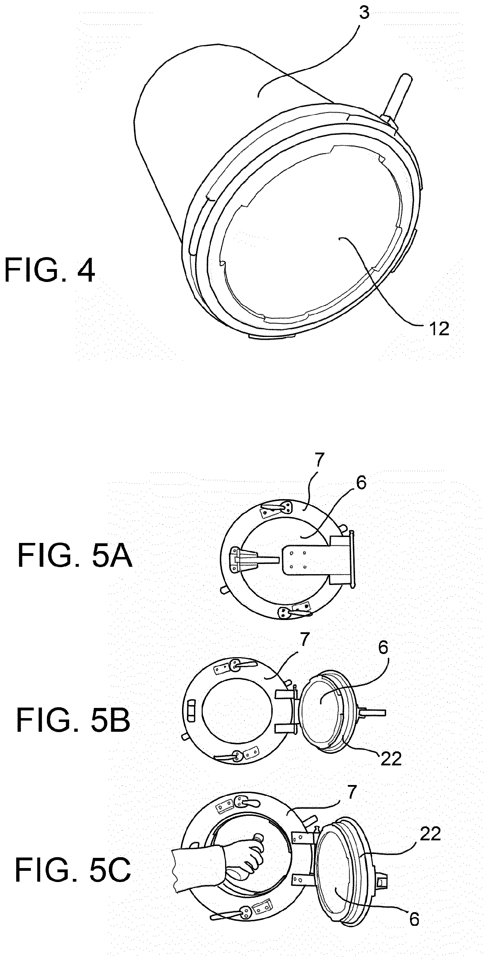

Furthermore, the container seal 10 and the door seal 22 are configured to tightly geometrically connect to each other (figs. 5A-5C).

Advantageously, the seals ensure tightness between the door flange 7 and the container lid 12 and the RTP door 6, thus further reducing the contamination risk.

According to an embodiment of the invention, the gyroscopic flask-holder 4 is rotatably connected to the RTP container 3 about a gyroscopic rotation axis 19.

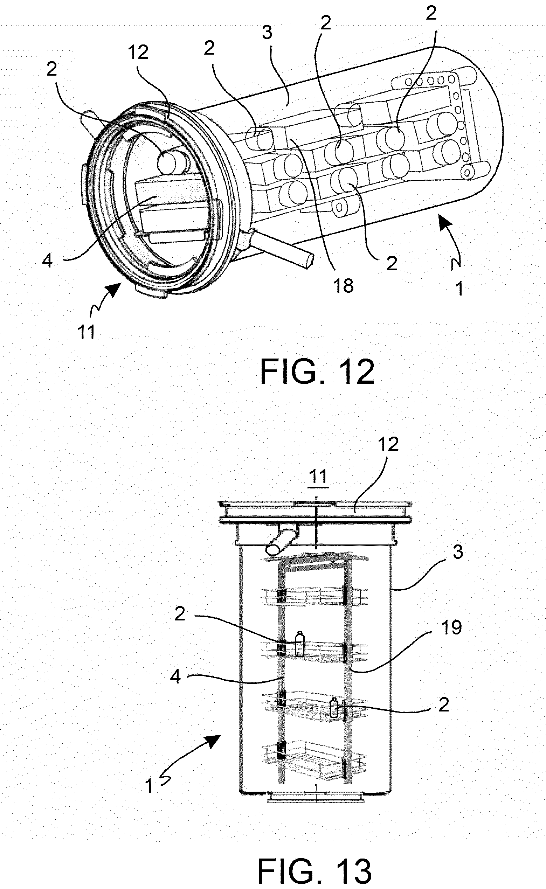

Furthermore, the gyroscopic flask-holder 4 comprises a flask-holder shelf 18 configured to support the biological samples 2 in a transverse position with respect to the gyroscopic rotation axis 19 (fig. 12).

According to an alternative embodiment, the gyroscopic flask-holder 4 comprises a flask-holder basket 19 configured to support the biological samples 2 in a parallel position with respect to the gyroscopic rotation axis 19 (fig. 13).

Advantageously, with the transfer system 1 for biological samples according to the present invention, the need to design an expensive and technically complex device, which implements therein the plurality of functions independently performed by the Grade A isolators (ISO 4.8), the standard external CO₂ incubators, and the transfer system 1 for biological samples, is avoided.

According to a further aspect of the invention, a transfer assembly 20 for biological samples 2 comprises a transfer system 1 for biological samples 2 as previously described.

Furthermore, the assembly 20 comprises a Grade A isolator 8, defining a door flange 7 with which an RTP door 6 is associated, and where the door flange 7 defines a door opening 13 which is closable by the RTP door 6.

Furthermore, the assembly 20 comprises at least one standard external CO₂ incubator 21. Preferably, the assembly 20 comprises a plurality of standard external CO₂ incubators 21.

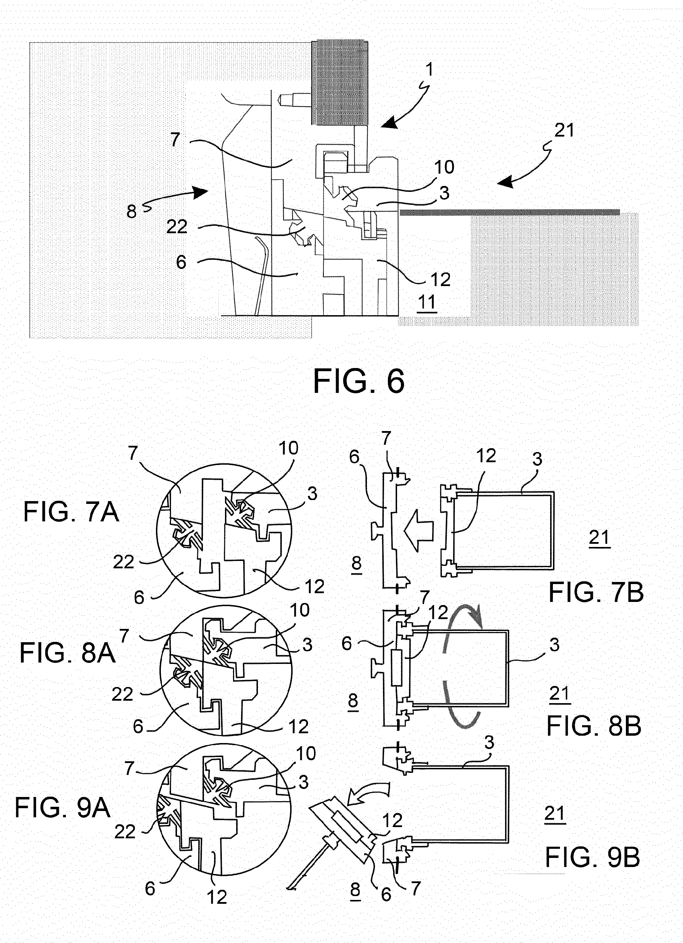

The container lid 12 and the RTP door 6 are configured to geometrically connect to each other, and where the geometrically connected container lid 12 and the RTP door 6 are jointly separable from the door flange 7 (figs. 6-9B).

Furthermore, the system 1 is configured to be inserted into standard external CO₂ incubators 21 (fig. 3).

Advantageously, such an assembly 20 achieves a transfer of biological samples from a Grade A isolator (ISO 4.8) to standard external CO₂ incubators, and vice versa, without breaking the isolation continuity of the biological samples.

According to a further aspect of the invention, a method of transferring biological samples 2 from a Grade A isolator 8 to standard external CO₂ incubators 21, where the Grade A isolator 8 defines a door flange 7 with which an RTP door 6 is associated, and where the door flange 7 defines a door opening 13 which is closable by the RTP door 6; comprises the steps of:
- providing a system 1 for transferring biological samples 2, as previously described (fig. 4);
- geometrically connecting the container lid 12 to the RTP door 6 (figs. 7A-8A);
- separating the geometrically connected container lid 12 and RTP door 6 from the door flange 7 (figs. 8B-9B);
- inserting at least one biological sample 2 into the RTP container 3 (figs. 5A-5C);
- repositioning the geometrically connected container lid 12 and RTP door 6 at the door flange 7;
- disconnecting the container lid 12 from the RTP door 6, so as to separate the RTP container 3 from the Grade A isolator 8;
- transferring the transfer system 1 for biological samples with the at least one biological sample from the Grade A isolator 8 to the standard external CO₂ incubator 21;
- inserting the RTP 3 container into the standard external CO₂ incubator 21;
- achieving an exchange of gas permeating the filter 5 and the auxiliary filter 9, between the RTP container 3 and the standard external CO₂ incubators 21 (fig. 3).

The transfer step of the transfer system 1 for biological samples from the Grade A isolator 8 to the standard external CO₂ incubators 21 can be conducted, for example, by an operator or by means of automated systems.

Advantageously, such a method achieves a transfer of biological samples from a Grade A isolator (ISO 4.8) to standard external CO₂ incubators, and vice versa, without breaking the isolation continuity of the biological samples. The permeability of the filter 5 and the auxiliary filter 9, HEPA or ULPA, to the gases inside the standard external CO₂ incubators allows the growth of the cells of the biological samples placed inside the transfer containers for the biological samples themselves. At the same time, such HEPA or ULPA filters prevent any contamination of the biological samples placed inside the transfer containers during the transfer from the Grade A isolator 8 to the standard external CO₂ incubators 21.

With further advantage, the gyroscopic flask-holder 4 keeps the orientation of the biological samples stable during the step in which the RTP container 3 is connected and disconnected from the RTP door 6.

Obviously, those skilled in the art will be able to make modifications or adaptations to the present invention without departing from the scope of the claims set forth hereinafter.

## Claims

1. A transfer system (1) for biological samples (2) comprising:
- an RTP container (3) defining a container opening (11) which is closable by a container lid (12);
- a HEPA or ULPA filter (5), positioned at the container lid (12), wherein said filter (5) is configured to:
a) protect said biological samples (2) from external contamination during the transfer of the RTP container (3) from a Grade A isolator to standard external CO₂ incubators;
b) protect said biological samples (2) from cross contamination when the RTP container (3) is inserted into standard external CO₂ incubators;
c) allow a gas exchange through the RTP container (3) inserted into standard external CO₂ incubators;
- an RTP door (6) associated with a door flange (7) of a Grade A isolator (8), wherein the door flange (7) defines a door opening (13) which is closable by the RTP door (6), wherein the container lid (12) and the RTP door (6) are configured to geometrically connect to each other, and wherein the geometrically connected container lid (12) and RTP door (6) are jointly separable from the door flange (7), and a gyroscopic flask-holder (4), wherein the gyroscopic flask-holder (4) is housed inside the RTP container (3).

2. A system (1) according to claim 1, wherein the RTP container (3) comprises an auxiliary container opening (15) which is closable by an auxiliary container lid (16) and opposite to the container opening (11).

3. A system (1) according to claim 2, comprising an auxiliary HEPA or ULPA filter (9) positioned at the auxiliary container lid (16), wherein said auxiliary filter (9) is configured to:
- protect said biological samples (2) from external contamination during the transfer of the RTP container (3) from a Grade A isolator to standard external CO₂ incubators;
- protect said biological samples (2) from cross contamination when the RTP container (3) is inserted into standard external CO₂ incubators;
- allow a gas exchange through the RTP container (3) inserted into standard external CO₂ incubators.

4. A system (1) according to claim 3, wherein the filter (5) and/or the auxiliary filter (9) is a HEPA H14 filter, or an ULPA U15 filter.

5. A system (1) according to any one of the preceding claims, wherein the RTP container (3) comprises a container seal (10) positioned at the container lid (12), and the RTP door (6) comprises a door seal (22), wherein the container seal (10) and the door seal (22) are configured to tightly geometrically connect to each other.

6. A system (1) according to any one of the preceding claims, wherein the gyroscopic flask-holder (4) is rotatably connected to the RTP container (3) about a gyroscopic rotation axis (19),
and wherein the gyroscopic flask-holder (4) comprises:
- a flask-holder shelf (18) configured to support the biological samples (2) in a transverse position with respect to the gyroscopic rotation axis (19), or
- a flask-holder basket (19) configured to support the biological samples (2) in a parallel position with respect to the gyroscopic rotation axis (19).

7. A transfer assembly (20) for biological samples (2), comprising:
- a transfer system (1) for biological samples (2) according to one of claims 1 to 6;
- a Grade A isolator (8), defining a door flange (7) with which an RTP door (6) is associated, and wherein the door flange (7) defines a door opening (13) which is closable by the RTP door (6);
- one or more standard external CO₂ incubators (21),
wherein the container lid (12) and the RTP door (6) are configured to geometrically connect to each other, and wherein the geometrically connected container lid (12) and RTP door (6) are jointly separable from the door flange (7),
and wherein the system (1) is configured to be insertable into one or more standard external CO₂ incubators (21).

8. A method of transferring biological samples (2) from a Grade A isolator (8) to one or more standard external CO₂ incubators (21), wherein the Grade A isolator (8) defines a door flange (7) with which an RTP door (6) is associated, and wherein the door flange (7) defines a door opening (13) which is closable by the RTP door (6);
said method comprising the steps of:
- providing a transfer system (1) for biological samples (2) according to one of claims 1 to 6;
- geometrically connecting the container lid (12) to the RTP door (6);
- separating the geometrically connected container lid (12) and RTP door (6) from the door flange (7);
- inserting at least one biological sample (2) into the RTP container (3);
- repositioning the geometrically connected container lid (12) and RTP door (6) at the door flange (7);
- disconnecting the container lid (12) from the RTP door (6), so as to separate the RTP container (3) from the Grade A isolator (8);
- transferring the transfer system (1) for biological samples with the at least one biological sample from the Grade A isolator (8) to the standard external CO₂ incubator (21);
- inserting the RTP container (3) into one or more standard external CO₂ incubators (21);
- achieving an exchange of gas permeating the filter (5) and the auxiliary filter (9), between the RTP container (3) and the one or more standard external CO₂ incubators (21).
